# FASCICULE DE BREVET EUROPEEN

(11) **EP 2 566 506 B1**
(45) Date de publication et mention de la délivrance du brevet: **06.07.2016**
(21) Numéro de dépôt: 10723638.2
(22) Date de dépôt: 07.05.2010
(51) Int. Cl.: A61K 39/008

(54) **COMPOSITION VACCINALE DESTINEE A LA PREVENTION OU AU TRAITEMENT DES LEISHMANIOSES**
ZUSAMMENSETZUNG EINES IMPFSTOFFS ZUR VERHINDERUNG ODER BEHANDLUNG VON LEISHMANIOSE
VACCINE COMPOSITION FOR PREVENTING OR TREATING LEISHMANIASES

(43) Date de publication de la demande: 13.03.2013
(73) Titulaire: VIRBAC, 06516 Carros (FR)
(72) Inventeur: RICHIER, Eric, F-06390 Coaraze (FR); LEBREUX, Bernard, F-06670 Saint Martin du Var (FR); BLOND, Denis, F-06510 Gattières (FR)
(74) Mandataire: Gevers & Orès
(86) Numéro de dépôt international: PCT/FR2010/000356
(87) Numéro de publication internationale: WO 2011/138513

(56) Documents cités:
- WO-A-99/11241
- WO-A-2007/003384
- FR-A- 2 825 633
- LEMESRE ET AL: "Long-lasting protection against canine visceral leishmaniasis using the LiESAp-MDP vaccine in endemic areas of France: Double-blind randomised efficacy field trial" VACCINE, BUTTERWORTH SCIENTIFIC. GUILDFORD, GB, vol. 25, no. 21, 27 avril 2007 (2007-04-27), pages 4223-4234, XP022050489 ISSN: 0264-410X
- BOURDOISEAU G ET AL: "Effective humoral and cellular immunoprotective responses in Li ESAp-MDP vaccinated protected dogs" VETERINARY IMMUNOLOGY AND IMMUNOPATHOLOGY, vol. 128, no. 1-3, Sp. Iss. SI, mars 2009 (2009-03), pages 71-78, XP002554451 ISSN: 0165-2427 cité dans la demande
- LEMESRE J-L ET AL: "Protection against experimental visceral leishmaniasis infection in dogs immunized with purified excreted secreted antigens of Leishmania infantum promastigotes" VACCINE, BUTTERWORTH SCIENTIFIC. GUILDFORD, GB, vol. 23, no. 22, 22 avril 2005 (2005-04-22), pages 2825-2840, XP004797054 ISSN: 0264-410X
- PALATNIK-DE-SOUSA CLARISA B ET AL: "FML vaccine against canine visceral leishmaniasis: from second-generation to synthetic vaccine." EXPERT REVIEW OF VACCINES AUG 2008, vol. 7, no. 6, août 2008 (2008-08), pages 833-851, XP009125352 ISSN: 1744-8395
- OLIVEIRA-FREITAS E ET AL: "Acylated and deacylated saponins of Quillaja saponaria mixture as adjuvants for the FML-vaccine against visceral leishmaniasis" VACCINE, BUTTERWORTH SCIENTIFIC. GUILDFORD, GB, vol. 24, no. 18, 1 mai 2006 (2006-05-01), pages 3909-3920, XP025151486 ISSN: 0264-410X [extrait le 2006-05-01]
- REED STEVEN G ET AL: "New horizons in adjuvants for vaccine development" TRENDS IN IMMUNOLOGY, vol. 30, no. 1, janvier 2009 (2009-01), pages 23-32, XP002554452 ISSN: 1471-4906
- PARRA ET AL: "Safety trial using the Leishmune<(>R) vaccine against canine visceral leishmaniasis in Brazil" VACCINE, BUTTERWORTH SCIENTIFIC. GUILDFORD, GB, vol. 25, no. 12, 13 février 2007 (2007-02-13), pages 2180-2186, XP005886437 ISSN: 0264-410X
- Petrovsky et al.: "New-Age Vaccine Adjuvants: Friend or Foe?", BioPharm International , 2 August 2007 (2007-08-02), pages 1-12, Retrieved from the Internet: URL:http://www.biopharminternational.com/b iopharm/Downstream+Processing/New-Age-Vacc ine-Adjuvants-Friend-or-Foe/ArticleStandar d/Article/detail/444996 [retrieved on 2014-09-18]
- FOGLIA MANZILLO VALENTINA ET AL: "Prospective study on the incidence and progression of clinical signs in naïve dogs naturally infected by Leishmania infantum.", PLOS NEGLECTED TROPICAL DISEASES 2013, vol. 7, no. 5, 2013, page e2225, ISSN: 1935-2735
- Oliva et al., Evidence for protection against active infection and disease progression in naïve dogs vaccinated with LiESP/QA-21 (CaniLeish) exposed to two consecutive Leishmania infantum transmission seasons // Proceedings of the WSAVA/FE-CAVA/BSAVA World Congress, Birmingham (UK), 11-15 April 2012

## Description

La présente invention concerne une composition vaccinale, à base de protéines d'excrétion-sécrétion issues de promastigotes et/ou d'amastigotes de *Leishmania* sp., destinée à la prévention ou au traitement des leishmanioses chez les canidés.

Les leishmanioses sont des infections parasitaires endémiques provoquées par un protozoaire flagellé de la famille des Trypanosomidae et du genre *Leishmania,* transmis par un phlébotome (voir pour revue : Chang, Leishmaniases, Encyclopedia of Life Sciences, 2005, John Wiley & Sons, Ltd. ; Bates, Leishmania, Encyclopedia of Life Sciences, 2006, John Wiley & Sons, Ltd.). De très nombreuses espèces de mammifères, comprenant l'Homme et le chien, sont infectées par ce parasite. Les phlébotomes femelles transmettent à l'hôte mammifère, au moment de la piqûre, le parasite sous sa forme flagellée infectante, le promastigote métacyclique. Les promastigotes métacycliques sont alors phagocytés par les macrophages présents dans le derme et se transforment en une forme non flagellée appelée amastigote.

Onze espèces de *Leishmania,* réparties en deux sous-genres, *Leishmania Leishmania* et *Leishmania Viannia,* sont d'importance médicale chez l'Homme. Chaque espèce de *Leishmania* est responsable d'une manifestation clinique bien définie : viscérale, cutanée, cutanée diffuse et cutanéo-muqueuse, selon que le parasite infecte les phagocytes mononucléés des viscères, du derme ou des muqueuses.

La leishmaniose est endémique dans 88 pays (dont l'Inde, le Bangladesh, le Népal, le Soudan et le Brésil), menaçant environ 350 millions de personnes. La leishmaniose viscérale, due à *Leishmania (L) donovani et Leishmania (L) infantum* (ou *L. chagasi),* affecte 500 000 personnes chaque année dans le monde. Elle représente la forme la plus sévère des leishmanioses.

Dans les zones touchées par la leishmaniose viscérale due à *L. infantum,* notamment sur le pourtour méditerranéen, les chiens, massivement infectés, représentent un réservoir de parasites pour l'Homme.

Chez l'Homme, les leishmanioses sont principalement traitées par des dérivés d'antimoine : l'antimoniate de méglumine (Glucantime®) et le stibogluconate de sodium (*Pentostam*®). Cependant, dans certaines régions du monde, le parasite est devenu résistant à l'antimoine. L'amphotéricine B (*Ambisome*®) y est alors maintenant utilisée pour le traitement de la leishmaniose viscérale ou cutanéo-muqueuse.

En revanche, il n'existe pour le moment aucun vaccin ni médicament prophylactique pour prévenir les leishmanioses chez l'Homme. En effet, la mise au point d'une composition vaccinale reste encore aujourd'hui problématique.

Plusieurs compositions vaccinales ont été proposées pour protéger l'Homme et/ou les chiens de la leishmaniose :
- une composition vaccinale à base d'une fraction membranaire antigénique de *Leishmania donovani* (dénommée antigène FML, *«fucose mannose ligand*») et d'un adjuvant tel que l'alum (adjuvant à base d'aluminium), le FIA (Adjuvant Incomplet de Freund), le BCG (Bacillus Calmette Guérin), l'interleukine 12, les mélanges de saponines tels que Riedel-de Haën saponin R (Sigma-Aldrich) ou Quil A (Brenntag) et la saponine purifiée QS21 (voir pour revue : Palatnik-de-Sousa et al., Expert. Rev. Vaccines, 2008, 7:833-851). La fraction membranaire antigénique FML comprend une glycoprotéine GP36 glycosylée ainsi que plusieurs protéines qui n'ont pas toutes été caractérisées.
- une composition vaccinale à base de protéines d'excrétion-sécrétion issues de promastigotes et/ou d'amastigotes de *Leishmania* sp. (ESPs) et de MDP (muramyl dipeptide) comme adjuvant induisant une réponse immunitaire de type Th1 (Bourdoiseau et al., Vet. Immunol. Immunopathol., 2009, 128 :71-78 ; Lemesre et al., Vaccine, 2007, 25:4223-4234 ; Lemesre et al., Vaccine, 2005, 23:2825-2840 ; Demandes Internationales n° WO 94/26899 et WO 2002/100430) ;
- une composition vaccinale comprenant deux peptides (A16E et A16G) issus de *Leishmania* et/ou leurs dérivés et un adjuvant tel que le MDP (Demande Internationale WO 03/025012) ou le QS21. Cette composition présente cependant le désavantage d'induire une immunité dirigée contre un nombre limité d'antigènes ;
- une composition vaccinale à base de promastigotes de *Leishmania braziliensis* inactivés par ultrasons et du mélange de saponines Riedel-de Haën saponin R comme adjuvant (Giunchetti et al., Vaccine, 2007, 25:7674-7686 et Giunchetti et al., Vaccine, 2008, 26:623-638 ; Demande de Brevet brésilien PI 0601225-6). Cette composition présente cependant l'inconvénient de contenir un mélange d'antigènes non caractérisés pouvant poser des problèmes d'innocuité et dont certains peuvent induire des réponses immunitaires de type Th1 et d'autres de type Th2.
- une composition vaccinale comprenant la protéine A2 recombinante provenant de la forme amastigote de *Leishmania donovani* et d'un adjuvant tel que le mélange de saponines Riedel-de Haën saponin R (Fernandes et al., Vaccine, 2008, 26:5888-5895, Demande Internationale WO 2008/009088).

Chez le chien, il existe actuellement deux vaccins commercialisés au Brésil : une composition vaccinale dénommée Leishmune®, constituée de l'antigène FML, du mélange de saponines Riedel-de Haën saponin R et de NaCl (Demande Internationale n° WO 2006/122382 ; Palatnik-de-Sousa *et al.,* 2008, cité ci-dessus), et une composition vaccinale dénommée LeishTec®, constituée de l'antigène A2 et d'un mélange de saponines.

Il ressort des nombreuses études portant sur la réponse immunitaire des hôtes (notamment chez le chien) lors de l'infection par *Leishmania,* que l'efficacité d'un candidat vaccin potentiel doit se traduire par l'activation chez l'hôte d'une réponse immunitaire de type Th1 avec production d'interféron gamma (Barbiéri, Parasite Immunol., 2006, 28:329-337, Revue; Alvar et al., Adv. Parasitol., 2004, 57:1-88 ; Ravindran et Ali, Curr. Mol. Med., 2004, 4:697-709 et Gradoni, Vet. Parasitol., 2001, 100:87-103). De nombreux adjuvants, tels que la saponine purifiée QS21, sont capables de stimuler une réponse immunitaire de type Th1.

Les saponines de type Quillaja sont un mélange de glycosides de triterpènes extraits de l'écorce de l'arbre Quillaja saponaria. Ce sont des produits naturels qui sont caractérisés par un certain nombre de propriétés communes, notamment l'aptitude à produire de la mousse en solution aqueuse. Des caractéristiques supplémentaires sont l'activité hémolytique, la toxicité vis-à-vis des poissons, la formation de complexes avec 1e cholestérol et dans certains cas une activité antibiotique. (Kofier, Die Saponine (Spri nger verlag), Berlin, 1927 ; Tschesche et coll., Chemie und Biologie der Saponine. Fortscher. 30 Chem. Org. Naturst. XXX'Abl (1972)).

Les mélanges de saponines de Quillaja disponibles sur le marché sont des mélanges bruts qui, à cause de leur variabilité et de leur absence de caractérisation complète, ne sont pas souhaitables pour une utilisation en pratique vétérinaire ou dans des compositions pharmaceutiques pour l'homme. Les impuretés présentes dans les produits disponibles sur le marché peuvent entraîner des réactions indésirables. De plus, la teneur en substance active d'un lot à l'autre de mélanges de saponines peut varier, ce qui diminue la reproductibilité.

Des adjuvants saponines ont été identifiés et purifiés par chromatographie liquide à haute pression (HPLC). La méthode de production de ces fractions purifiées est décrite dans le Brevet américain US 5,057,540.

Parmi elles, la saponine QA21 (ou QA-21) sensiblement pure est caractérisée par les propriétés suivantes :
- activité adjuvante immunitaire,
- contient environ 22% d'hydrates de carbone (analysé par l'anthrone) par poids sec,
- a un maximum d'absorption U.V. à 205-210 nm,
- a un temps de rétention d'environ 51 minutes par RP-HPLC sur une colonne Vydac C₄ ayant des particules de 5µm, des pores de 330 A, un diamètre interne de 4,6 mm et une longueur de 25 cm dans un solvant d'acide acétique à 40 mM dans du méthanol:eau (58:42; v:v) à un débit de 1 ml par minute,
- est éluée avec du méthanol à 69 à 70 % d'une colonne Vydac C₄ ayant des particules de 5µm, des pores de 330 A, un diamètre interne de 10 mm et une longueur de 25 cm dans un solvant d'acide acétique à 40 mM avec un gradient d'élution de 50 à 80 % de méthanol,
- a une concentration micellaire critique de 0,03 % (p:v) dans l'eau et de 0,02 % (p:v) dans du sérum tamponné phosphate,
- provoque l'hémolyse de globules rouges de moutons à des concentrations de 25 µg par ml ou davantage, et
- contient les monosaccharides rhamnose terminal, arabinose terminal, apiose terminal, xylose terminal, 4-rhamnose, glucose terminal, galactose terminal, 2-fucose, 3-xylose, 3,4-rhamnose et acide 2,3-glucuronique.

Le "terme sensiblement pur" signifie sensiblement exempt de composés normalement associés à la saponine dans son état naturel et provoquant une réponse chromatographique, des profils d'élution et une activité biologique constants et reproductibles.

La saponine QA21 sensiblement pure décrite précédemment est également dénommée QS21 (ou QS-21) (voir pour revue Kensil et al., Pharm. Biotechnol., 1995, 6:525-541 ; Kensil et Kammer, Expert Opin. Investig. Drugs., 1998, 7:1475-1482 et Soltysik et al., Vaccine, 1995, 13:1403-10). QS21 est une molécule amphipathique (soluble dans l'eau), présentant une masse molaire de 1988 g/mol. Sa formule développée est représentée à la Figure 1. Un procédé d'obtention de QS21 est décrit dans le Brevet américain US 5,057,540. Néanmoins, QS21 peut aussi être purifiée à partir des mélanges de saponines tels que Riedel-de Haën saponin R ou Quil A. L'efficacité de QS21 a été évaluée en immunologie humaine et vétérinaire, sous sa forme pure, pour le traitement de cancers, du SIDA ou de la malaria ou de la leucose féline (Vaccin Leucogen®- Virbac). Dans les mélanges de saponines Riedel-de-Haen saponin R et Quil A, QS21 représenterait la fraction saponine majoritaire, respectivement aux environs de 18% et 40-50% (Palatnik-de-Sousa *et al.,* 2008, cité ci-dessus et Parra et al, Vaccine, 2007, 25:2180-2186). La proportion en QS21 dans les mélanges de saponines Riedel-de Haën saponin R et Quil A varie cependant en fonction des publications : par exemple, dans le mélange Quil A, selon Kensil et al. (J. Immunol., 1991, 146:431-437), QS21 ne représente pas la fraction saponine majoritaire alors que selon d'autres publications des mêmes auteurs, elle représenterait 40% (Parra *et al.,* 2007, cité ci-dessus) ou 49% (Santos et al., 2002, Vaccine, 21, 30-43) du mélange. Cette variabilité en quantité de QS21 peut souligner une faible reproductibilité de la production de ces mélanges de saponines. QS21 induit une bonne réponse immunitaire de type Th1, mais présente cependant plusieurs effets secondaires, parmi lesquels on peut citer : des oedèmes au niveau du point d'injection due à une forte réaction inflammatoire locale, des douleurs locales, une anorexie, une apathie, des vomissements et des diarrhées. Ces effets secondaires en limitent donc son utilisation. Une solution proposée pour remédier à ces désavantages consiste à associer QS21 à des composés diminuant ses effets secondaires tels que, par exemple, un stérol (Demande Internationale WO 96/33739).

Les Inventeurs se sont donnés pour but de pourvoir à une nouvelle composition vaccinale destinée à la prévention ou au traitement des leishmanioses qui protégerait durablement les mammifères contre ces maladies, qui répond mieux aux besoins de la pratique que les compositions vaccinales proposées antérieurement et qui entraîne peu ou pas d'effets secondaires.

La présente invention a en conséquence pour objet une composition vaccinale destinée à la prévention ou au traitement de la leishmaniose telle que définie dans les revendications.

On entend par « prévention de la leishmaniose », l'induction d'une protection ou d'une réponse immunitaire contre des infections par *Leishmania.*

On entend par « traitement de la leishmaniose », la diminution des symptômes de la leishmaniose, ou la diminution ou suppression des *Leishmania* chez le mammifère infecté.

Lesdites protéines d'excrétion-sécrétion (ESPs) sont susceptibles d'être obtenues en mettant en oeuvre les procédés décrits dans les Demandes Internationales WO 94/26899 et WO 2002/100430.

Selon un mode de réalisation préféré de la présente invention, lesdites protéines d'excrétion-sécrétion sont issues de promastigotes et/ou d'amastigotes, de préférence de promastigotes seulement, de *Leishmania (L) infantum.*

La quantité de saponine QA21 sensiblement pure dans ladite composition vaccinale est de 60 µg/dose vaccinale.

On entend par « dose vaccinale », le volume de la composition vaccinale selon la présente invention administré à un mammifère. La dose vaccinale varie en fonction de la taille du mammifère auquel est administrée la composition vaccinale. Cette dose est généralement de 0,1 à 5 mL. De préférence, la dose vaccinale est de 1 mL.

De manière surprenante, les Inventeurs ont montré qu'une dose de QA21 de 60 µg par dose vaccinale présentait le meilleur rapport efficacité/innocuité pour les chiens traités. En effet, les réactions (effets secondaires) générales des animaux vaccinés avec cette dose étaient inexistantes et les réactions locales faibles. De plus, à cette dose, la réponse humorale (cinétique et titre) était conservée par rapport aux doses plus élevées. En outre, il n'est pas nécessaire d'associer QA21 à des composés, tels que des stérols comme préconisés dans l'art antérieur, pour en diminuer ses effets secondaires, ou de l'utiliser dans des mélanges de saponines tels que Riedel-de Haën saponin R ou Quil A, rendant ainsi la fabrication d'une composition vaccinale plus simple et contenant un adjuvant caractérisé, défini et reproductible, donc mieux maîtrisable, et induisant un rapport activité/innocuité constant dans le temps.

Selon un mode de réalisation avantageux de l'invention, la quantité d'ESPs dans ladite composition vaccinale est de 80 à 140 µg par dose vaccinale, particulièrement de 90 à 130 µg par dose vaccinale et plus préférentiellement de 110 µg par dose vaccinale.

Selon un autre mode de réalisation de l'invention, la saponine QA21 sensiblement pure est le seul adjuvant saponine de la composition.

Selon un mode de réalisation encore plus avantageux, la saponine QA21 sensiblement pure est le seul adjuvant de la composition. (Aucun autre élément de la composition ne peut adjuver les antigènes).

L'excipient pharmaceutiquement acceptable peut être tout excipient connu de l'Homme du métier. De préférence, il comprend ou est constitué d'un ou plusieurs des composés choisis dans le groupe constitué par le chlorure de sodium (NaCl), les agents de lyophilisation tels que le mannitol et le saccharose, les tampons tels que le Tris (trishydroxyméthylaminométhane), les conservateurs ou les stabilisants.

Selon l'invention, la composition vaccinale est destinée à la prévention ou au traitement de la leishmaniose chez un mammifère de l'espèce *Canis lupus* (le chien).

Selon un autre mode de réalisation avantageux de l'invention, ladite composition vaccinale est destinée à la prévention ou au traitement de la leishmaniose viscérale, plus particulièrement celle causée par *Leishmania (L) infantum, Leishmania (L) chagasi* et *Leishmania (L) donovani.*

Selon ce mode de réalisation, ladite composition vaccinale peut se présenter sous une forme lyophilisée.

La présente invention a aussi pour objet un procédé de fabrication d'une composition vaccinale destinée à la prévention ou au traitement de la leishmaniose chez un mammifère, tel que défini dans les revendications.

Selon le procédé de l'invention, la quantité de saponine QA21 sensiblement pure dans ladite composition est de 60 µg/dose vaccinale, et la quantité d'ESPs est de 80 à 140 µg/dose vaccinale, de préférence encore de 90 à 130 µg/dose vaccinale et plus préférentiellement de 110 µg/dose vaccinale.

Un kit comprenant au moins une dose injectable d'une composition vaccinale selon la présente invention est également décrit.

Une méthode pour traiter ou prévenir la leishmaniose chez un mammifère, comprenant l'administration audit mammifère d'une composition vaccinale telle que définie ci-dessus est également décrite. De préférence, l'administration de la composition vaccinale s'effectue par injection sous-cutanée.

Outre les dispositions qui précèdent, l'invention comprend encore d'autres dispositions, qui ressortiront de la description qui va suivre, qui se réfère à des exemples de mise en oeuvre de la composition vaccinale objet de la présente invention ainsi qu'aux dessins annexés, dans lesquels :

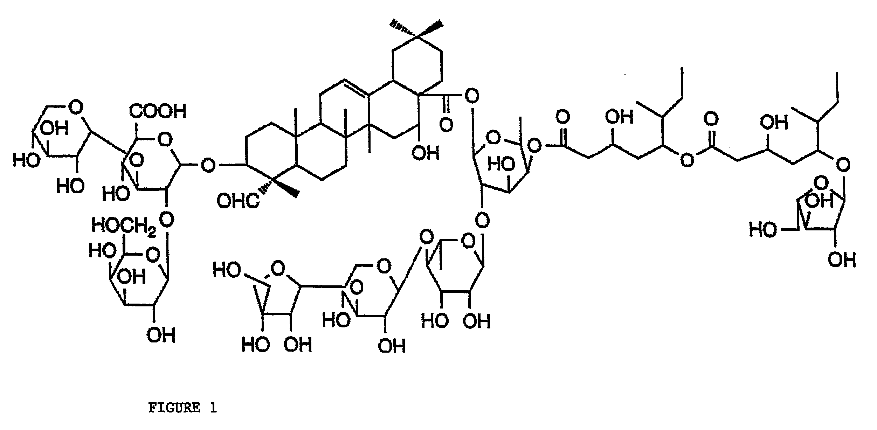

- la Figure 1 illustre la formule développée du QS21 ;
- la Figure 2 est un graphique représentant la moyenne des titres (en log10) d'IgG1 anti-ESPs en fonction du temps pour chaque groupe de chiens vaccinés avec 110 µg/dose de protéines d'excrétion-sécrétion issues de promastigotes de *L. infantum* (ESPs) et comme adjuvant une quantité de QA21 respectivement de 500 µg/dose (composition vaccinale LSH303), 250 µg/dose (composition vaccinale LSH304), 125 µg/dose (composition vaccinale LSH305) et 60 µg/dose (composition vaccinale LSH306) ;
- la Figure 3 est un graphique représentant la moyenne des titres (en log10) d'IgG2 anti-ESPs en fonction du temps pour chaque groupe de chiens vaccinés avec la composition vaccinale LSH303, LSH304, LSH305 ou LSH306 ;
- la Figure 4 est un graphique représentant les titres (en log10) d'IgG1 anti-ESPs en fonction du temps chez les deux chiens vaccinés avec la composition vaccinale LSH304 (Figure 4A) et chez les deux chiens vaccinés avec la composition vaccinale LSH306 (Figure 4B) ;
- la Figure 5 est un graphique représentant les titres (en log10) IgG2 anti-ESPs en fonction du temps chez les deux chiens vaccinés avec la composition vaccinale LSH304 (Figure 5A) et chez les deux chiens vaccinés avec la composition vaccinale LSH306 (Figure 5B) ;
- la Figure 6 est un graphique représentant, en fonction du temps, l'activité leishmanicide (pourcentage d'amastigotes de *L. infantum* tués) des macrophages des deux chiens vaccinés avec la composition vaccinale LSH304 (Figure 6A) et des deux chiens vaccinés avec la composition vaccinale LSH306 (Figure 6B) ;
- la Figure 7 est un graphique représentant la production de NO (nmol/10⁵ cellules/72h) en fonction du temps chez les deux chiens vaccinés avec la composition vaccinale LSH304 (Figure 7A) et chez les deux chiens vaccinés avec la composition vaccinale LSH306 (Figure 7B) ;
- la Figure 8 est un graphique représentant la quantité (en pourcentage) de l'enzyme NO-synthase inductible dans le surnageant des co-cultures de macrophages et de lymphocytes autologues provenant des deux chiens vaccinés avec la composition vaccinale LSH304 (Figure 8A) ou des deux chiens vaccinés avec la composition vaccinale LSH306 (Figure 8B).

### EXEMPLE 1 : Etude d'innocuité et d'efficacité chez le chien de 3 injections d'une composition vaccinale comprennent des ESPs de L. infantum et du QA21.

Le but de cette étude était de tester différentes concentrations de QA21 dans une composition vaccinale comprennant des ESPs de promastigotes de L. *infantum* et du QA21 comme seul adjuvant afin de déterminer le meilleur rapport efficacité/innocuité.

### 1.1. Matériels et Méthodes

### Obtentions des ESPs

Les protéines d'excrétion-sécrétion (ESPs) issues de promastigotes de *Leishmania infantum* produites dans un milieu axénique et asérique ont été obtenues conformément à la méthode décrite dans les Demandes Internationales WO 2002/100430 et WO 94/26899 en utilisant les milieux et paramètres de culture adaptés à une production industrielle.

### Compositions vaccinales

Des compositions vaccinales constituées de 110 µg/dose de protéines d'excrétion-sécrétion (ESPs) issues de promastigotes de *Leishmania infantum* produites dans un milieu axénique et asérique, de quantités décroissantes de QA21 : 500 µg/dose (composition vaccinale LSH303), 250 µg/dose (composition vaccinale LSH304), 125 µg/dose (composition vaccinale LSH305) et 60 µg/dose (composition vaccinale LSH306), et d'un excipient pharmaceutiquement acceptable (Excipient TRIS Mannitol Saccharose et du NaCl) ont été préparées.

Les chiens : 9 chiens de race Beagle âgés de 6 mois minimum, conventionnellement vaccinés et vermifugés une semaine avant la première vaccination avec la composition vaccinale à base d'ESPs et de QA21.

### Protocole de vaccination

2 chiens ont reçu chacun 3 injections de la composition vaccinale LSH303 à 4 semaines d'intervalle, à J0, 27 et 55 ;
2 chiens ont reçu chacun 3 injections de la composition vaccinale LSH304 à 4 semaines d'intervalle, à J0, 27 et 55 ;
2 chiens ont reçu chacun 3 injections de la composition vaccinale LSH305 à 4 semaines d'intervalle, à J0, 27 et 55 ;
3 chiens ont reçu chacun 3 injections de la composition vaccinale LSH306 à 4 semaines d'intervalle, à J0, 27 et 55.

Les vaccinations ont été réalisées par voie sous-cutanée.

### Critères d'évaluation de la réponse immunitaire

(i) *Suivi clinique local et général* : un suivi clinique local et général (incluant la mesure de la température rectale et du poids) a été effectué chaque jour de vaccination et, 1 et 3 jours après chaque vaccination. En cas de persistance des signes cliniques 3 jours après la vaccination, ce suivi a été effectué 2 fois par semaine jusqu'à la disparition complète des symptômes. Un examen clinique complet, consistant à vérifier l'état général des chiens et incluant entre autres la mesure de la température rectale et du poids, a été effectué une fois par semaine.
(ii) *Analyse sérologique* :
   Des échantillons de sang ont été régulièrement prélevés afin de mesurer la réponse sérologique des chiens. Le dosage des anticorps IgG1 et IgG2 anti-ESPs dans le sérum des chiens vaccinés a été effectué à l'aide de la méthode ELISA, (Enzyme Link Immunosorbent Assay), avec les anticorps de chèvre anti IgG1 de chien marqués à la peroxydase (Réf Bethyl laboratoire A40-120P), et les anticorps de mouton anti IgG2 de chien marqués à la peroxydase (Réf. Bethyl laboratoire A40-121P). L'évaluation de la réponse IgG1 et IgG2 contre les ESPs a été effectuée à l'aide d'un test Western Blot.

### 1.2. Résultats

### Examen clinique général

- pour le groupe de chiens qui a reçu la composition vaccinale LSH303 : un pic d'hyperthermie a été observé après la 2^{ème} vaccination (39,7°C et 40,8°C), et pour l'un d'entre eux aussi après la 3^{éme} vaccination (39,7°C). Ces chiens ne montraient aucun symptôme d'ordre général. Les pertes de poids observées dans ce groupe n'ont jamais dépassé 3% et sont compatibles avec les valeurs observées dans les 3 autres groupes.
- pour les groupes de chiens qui a reçu la composition vaccinale LSH304, LSH305 ou LSH306: aucun signe général n'a été observé après la vaccination. Les variations transitoires et ponctuelles de gain de poids observées dans ces groupes sont compatibles avec la courbe de croissance des chiens de cet âge et ne sont pas liées aux vaccinations. De légères augmentations de la température rectale ont parfois été observées 24 heures après l'injection du vaccin dans les groupes ayant reçu la composition LSH304 ou LSH306, mais ne sont pas significatives.

### Examen clinique local

- pour le groupe de chiens qui a reçu la composition vaccinale LSH303 : des réactions locales sévères ont été observées 24 heures après la vaccination, dont des tuméfactions de plus de 2 cm de diamètre, une douleur régulière au point d'injection (simple sensibilité lors de la palpation ou douleur persistante) et des cas d'oedème de grande surface. Ces signes avaient disparu en 7 jours maximum après l'injection de la composition vaccinale pour la douleur, en 6 jours maximum pour les oedèmes et en 17 jours maximum pour les gonflements.
- pour le groupe de chiens qui a reçu la composition vaccinale LSH304 : les réactions locales observées 24 heures après les vaccinations ont été des tuméfactions entre 1 et 2 cm de diamètre, et une sensibilité au point d'injection de la 2^{ème} vaccination (2 chiens) et de la 3^{ème} vaccination (1 chien). Un léger prurit (inférieur à 1 minute) a été observé immédiatement après la 3^{ème} vaccination (1 chien). Des oedèmes modérés (localisés) à sévères (de grandes surfaces) ont été observés, respectivement, au niveau du 2^{ème} et 3^{eme} point d'injection. Ces signes avaient disparu en 3 jours maximum après les injections pour la douleur, en 6 jours maximum pour les oedèmes et en 17 jours maximum pour les gonflements.
- pour le groupe de chiens qui a reçu la composition vaccinale LSH305 : les réactions locales observées 24 heures après la vaccination étaient similaires en intensité à celles observées pour le groupe de chiens ayant reçu la composition vaccinale LSH304, avec des tuméfactions entre 1 et 2 cm de diamètre, et une sensibilité au niveau du site d'injection de la 2^{eme} vaccination (1 chien). Des oedèmes modérés (localisés) à sévères (de grandes surfaces) ont également été observées au niveau du 3^{eme} point d'injection. Ces signes avaient disparu en 3 jours maximum après les injections pour la douleur, en 6 jours maximum pour les oedèmes et en 17 jours maximum pour les gonflements.
- pour le groupe de chiens qui a reçu la composition vaccinale LSH306 : Les signes cliniques sont restés bénins et se résumaient à 2 cas de prurit léger (inférieur à 1 minute) observés immédiatement après la 3^{eme} injection. Une tuméfaction jusqu'à 1-2 cm de diamètre a été observée 24 heures après la 1^{ere} et la 2^{ème} vaccination chez tous les chiens. Cette tuméfaction avait disparu en 13 jours maximum après l'injection. Seul un chien a aussi montré une tuméfaction pendant 24 heures après la 3^{eme} vaccination, qui avait disparu 6 jours après l'injection.

### Suivi sérologique

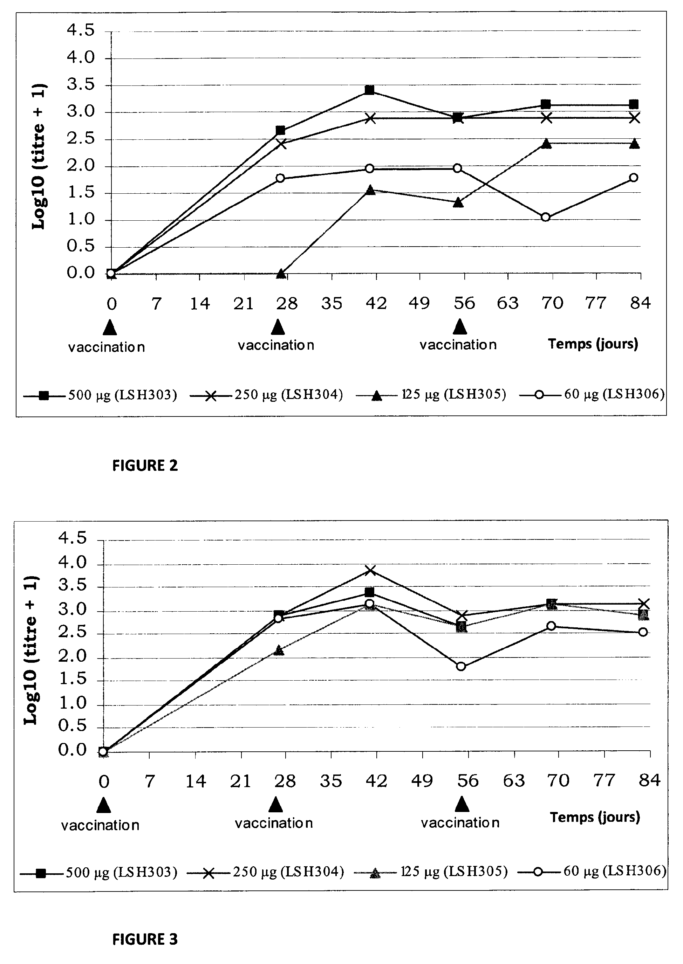

Le résultat du dosage des anticorps IgG1 et IgG2 anti-ESPs est présenté aux Figures 2 et 3 respectivement.

L'évolution de la moyenne des titres d'IgG1 et IgG2 anti-ESPs était similaire pour les groupes de chiens ayant reçu la composition vaccinale LSH303 ou LSH304 :
- les IgG1 ont atteint un titre moyen de 10^{3,13} et 10^{2,89} (titre en log10) deux semaines après la dernière injection, respectivement dans le groupe ayant reçu la composition vaccinale LSH303 et dans le groupe ayant reçu la composition vaccinale LSH304 ;
- les IgG2 ont atteint un titre moyen de 10^{3,13} (titre en log10) deux semaines après la dernière injection dans les deux groupes.

L'évolution de la moyenne des titres d'IgG1 et IgG2 anti-ESP a été plus faible pour les groupes de chiens ayant reçu la composition vaccinale LSH305 ou LSH306 :
- les IgG1 ont atteint un titre moyen de 10^{2,42} et 10^{1,04} (titre en log10) deux semaines après la dernière injection, respectivement dans le groupe ayant reçu la composition vaccinale LSH305 et dans le groupe ayant reçu la composition vaccinale LSH306 ;
- les IgG2 ont atteint un titre moyen de 10^{3,13} et 10^{2,65} (titre en log10) deux semaines après la dernière injection, respectivement dans le groupe ayant reçu la composition vaccinale LSH305 et dans le groupe ayant reçu la composition vaccinale LSH306.

La diminution de la réponse sérologique, observée avec les plus faibles concentrations d'adjuvant, est plus importante avec l'isotype IgG1.

Enfin, aucune augmentation significative de la réponse sérologique n'a été observée après la seconde injection, quelle que soit la composition vaccinale utilisée.

La réponse spécifique IgG1 et IgG2 contre les ESPs analysée par Western Blot a révélé une séroconversion de 100% dans les 4 groupes de chiens.

### Conclusion

L'innocuité générale d'une composition vaccinale selon la présente invention formulée avec 60 µg/dose de QA21 est bonne. En revanche, des oedèmes et des douleurs ont été observés avec les compositions vaccinales formulées avec 500, 250 et 125 µg/dose de QA21.

Les données sur l'innocuité locale sont en faveur d'une quantité de 60 µg/dose de QA21 ; quantité pour laquelle il est seulement observé de légères tuméfactions durant moins de 13 jours.

Le suivi sérologique montre un maintien des anticorps IgG2 anti-ESPs et une diminution des anticorps IgG21 anti-ESPs pour les concentrations de QA21 les plus faibles : 60 et 125 µg/dose.

Une dose de 60 µg de QA21 induit donc des réactions locales très modérées tout en maintenant une réponse humorale élevée et présente en conséquence le meilleur rapport efficacité/innocuité pour les chiens traités.

### EXEMPLE 2 : Etude d'innocuité et d'efficacité chez le chien de deux injections d'un vaccin composé d'ESPs de L infantum et de QA21 comme adjuvant ; analyse de la réponse immunitaire

Le but de cette étude était de comparer l'efficacité et l'innocuité de deux formulations de vaccin à base d'ESPs de promastigotes de *L infantum* et contenant des quantités différentes de QA21 (60 µg/dose ou 250 µg/dose). L'efficacité a été étudiée par l'analyse de la réponse immunitaire humorale et cellulaire. L'innocuité a été étudiée par l'évaluation des réactions locales et générales.

### 2.1. Matériels et Méthodes

Compositions vaccinales : LSH304 et LSH306 (voir Exemple 4.1). Les chiens : 6 chiens SPF âgés de 21 semaines.

### Protocole de vaccination :

2 chiens ont reçu chacun 2 injections de la composition vaccinale LSH304 à 4 semaines d'intervalle, à J0 et J28 ;
2 chiens ont reçu chacun 2 injections de la composition vaccinale LSH306 à 4 semaines d'intervalle, à J0 et J28 ;
2 chiens n'ont pas été vaccinés.

Les vaccinations ont été réalisées par voie sous-cutanée.

### Préparation de la leishmanine

Une souche de *Leishmania infantum* a été décongelée à J0 et mise en culture. Après 5 à 6 jours de culture une amplification a été réalisée par augmentation du volume de culture. Les parasites ont été maintenus en culture jusqu'à atteindre la phase stationnaire de croissance. Les parasites ont alors été récoltés, la suspension a été lavée trois fois avec du PBS. Les parasites ont été lysés par 3 cycles de congélation/décongélation. La leishmanine ainsi obtenue a été conservée à ≤ -70°C.

### Test DTH

Le test DTH cutané (*Delayed Type Hypersensibility*), bien connu de l'Homme du métier, permet de déterminer si une réponse à médiation cellulaire est de type Th1 ou Th2 (Black C.A., Dermatol. Online J., 1999, 5:7 http://dermatology.cdlib.org/DOJvol5num1/reviews/black.html ; Kid P., Alternative Medicine Review, 2003, 8:223-246).

Quatre semaines après la dernière injection (J56), chaque chien a reçu par voie intradermique 0,1 ml de leishmanine dans le côté droit du museau et de 0,1 ml de solution de contrôle (0,9% de NaCl et 0,4% de phénol) dans le côté gauche du museau.

### Evaluation de l'activité leishmanicide :

Les cellules mononuclées du sang périphérique ou PBMC sont obtenues par centrifugation sur un gradient de Ficoll, puis les monocytes sont séparés de la population lymphocytaire par adhérence sur plastique, et chaque population est incubée à 37°C afin de permettre aux monocytes de devenir des macrophages matures. Les macrophages ainsi obtenus sont alors infectés avec des parasites Leishmanie. Après l'incubation, l'excès de parasites est éliminé puis les macrophages sont incubés pendant 24h dans du milieu complet. Les lymphocytes autologues sont alors ajoutés à la moitié des puits afin d'évaluer la capacité des macrophages à tuer les parasites après activation par les lymphocytes. Les puits sans lymphocytes servent de contrôles de multiplication du parasite. Après 72h, les surnageants de culture sont récupérés et conservés, les lymphocytes sont éliminés et les macrophages sont fixés dans les puits au méthanol. Une partie des macrophages est colorée au Giemsa et le pourcentage d'inhibition de l'index parasitaire est évalué. L'autre partie des macrophages est utilisée pour la détection de la NO synthase inductible (iNOS) en utilisant des anticorps spécifiques ou/et par immunomarquage avec la technique Avidin-Biotin/peroxidase-Complex. La production de NO2 (impliqué dans la cascade du NO) est évaluée dans les surnageants de culture à l'aide de la technique de référence de Griess modifiée

### Critères d'évaluation de la réponse immunitaire :

Durant la « phase de vaccination » : les réactions générales et locales ont été évaluées de J0 à J4 et de J28 à J32, comme décrit à l'Exemple 4.1 ; l'analyse sérologique a été effectuée comme décrit à l'Exemple 4.1 ; l'activité leishmanicide des macrophages a été mesurée; les cellules T CD4⁺CD5⁺ et CD8⁺CD5⁺, et les cellules B ont été dénombrées par une méthode de cytométrie en flux.

Durant la « phase DTH » (suite à l'injection de la leishmanine) : la réaction générale a été évaluée tous les jours de J56 à J63 ; les réactions locales ont été mesurées tous les jours avec un pied à coulisse ; l'analyse sérologique a été effectuée par la méthode ELISA et par Western Blot comme décrits à l'Exemple 4.1 ; l'activité leishmanicide des macrophages a été mesurée ; les cellules T CD4⁺CD5⁺ et CD8⁺CD5⁺, et les cellules B ont été dénombrées.

### 2.2. Résultats

### 2.2.1 Résultats durant la « phase de vaccination »

### Examen clinique général

L'un des deux chiens vaccinés avec la composition vaccinale LSH304 (contenant une dose de 250 µg de QA21) a été atteint d'hyperthermie pendant un jour, associée à une dépression pendant 2 jours, après la 1^{ère} vaccination. Les deux chiens ont montré une hypertrophie des ganglions lymphatiques poplités et sous-capsulaires après la 2^{nd} vaccination ; cette hypertrophie a disparu en 4 jours. Les deux chiens vaccinés avec la composition vaccinale LSH306 (contenant une dose de 60 µg de QA21) ont seulement montré une légère hypertrophie des ganglions lymphatiques poplités et sous-capsulaires après la 2^{nd} vaccination, qui a disparu en 4 jours. Tous les animaux ont montré un gain de poids de 15% et 37% entre J0 et J56.

### Examen clinique local

Des réactions locales de différentes intensités ont été observées dans les deux groupes de chiens vaccinés :
- dans le groupe de chiens vaccinés avec la composition vaccinale LSH304: Observation d'un oedème de 3 à 4 cm de diamètre associé à une douleur après la 1^{ère} vaccination. Pour un des deux chiens, l'oedème a évolué en un nodule (1 cm) qui a disparu en 16 jours. Observation d'un oedème déclive sévère de 5 à 10 cm de diamètre après la 2^{nd} vaccination, qui a disparu en 4 jours.
- dans le groupe de chiens vaccinés avec la composition vaccinale LSH306: Observation d'une tuméfaction inférieur à 1,5 cm de diamètre après la 1^{ére} vaccination, qui a disparu 1 jour après. Pour un des deux chiens, observation d'un oedème déclive de 3 à 5 cm de diamètre après la 2^{nd} vaccination, qui a disparu en 3 jours.

### Analyse sérologique

Tous les chiens étaient séronégatifs pour les anticorps anti-ESPs avant la 1^{ere} vaccination.

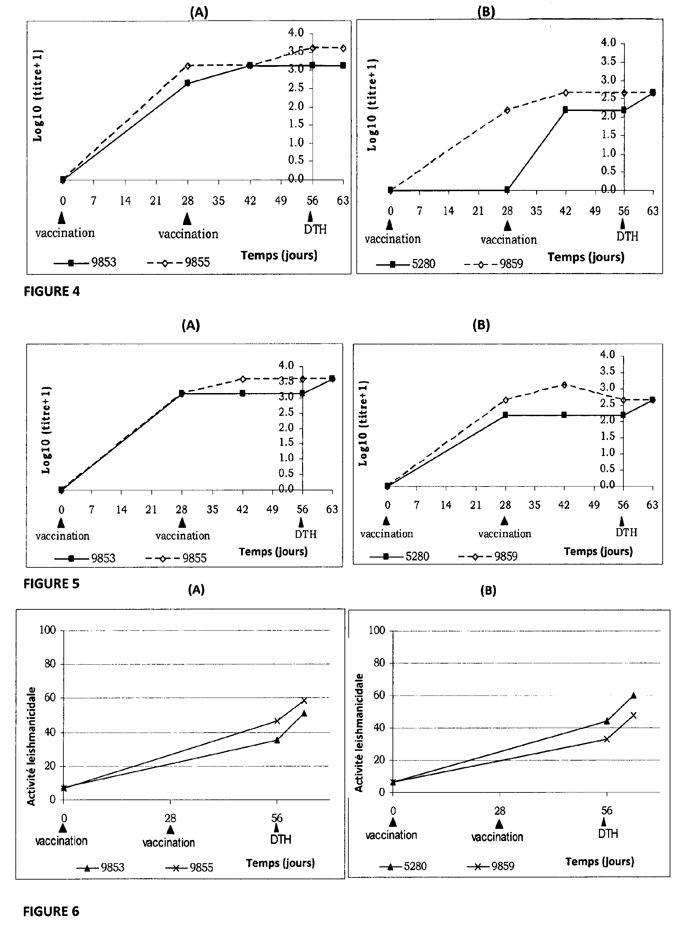

Les chiens vaccinés avec la composition vaccinale LSH304 présentaient des titres élevés d'anticorps IgG1 et IgG2 anti-ESPs (voir Figures 4A et 4B). La séroconversion IgG1 et IgG2 a eu lieu à partir de la 1^{ere} vaccination.

Les chiens vaccinés avec la composition vaccinale LSH306 présentaient des titres plus faibles en anticorps IgG1 et IgG2 anti-ESPs (voir Figure 5A et 5B). Pour l'un des deux chiens, la séroconversion en IgG2 a eu lieu à partir de la 1^{ère} vaccination alors que la séroconversion en IgG1 est apparue après la 2^{nd} vaccination. Pour l'autre chien, la séroconversion pour les deux sérotypes a eu lieu après la 1^{ere} vaccination.

Les Figures 4 et 5 montrent aussi que les titres d'anticorps IgG1 et IgG2 sont corrélés avec la dose de l'adjuvant dans la composition vaccinale.

Pour les deux groupes de chiens, les anticorps IgG2 dirigés contre les ESPs, révélés par la méthode Western Blot, sont apparus à partir de la 1^{ère} vaccination alors que les anticorps IgG1 sont apparus à partir de la 2^{nd} vaccination.

### Activité leishmanicide des macrophages

Tous les chiens étaient négatifs pour l'activité leishmanicide des macrophages avant la 1^{ère} vaccination. Les macrophages des chiens des deux groupes vaccinés étaient capables de tuer les amastigotes de *L. infantum* après la 2^{nd} vaccination quand ils étaient co-cultivés avec des lymphocytes autologues. Le niveau de l'activité leishmanicide des macrophages était similaire dans les deux groupes vaccinés (Figures 6A et 6B). En revanche, aucune activité leishmanicide des macrophages des chiens du groupe contrôle n'a été mesurée au cours de l'étude.

Afin de déterminer si le monoxyde d'azote (NO) jouait un rôle dans la mort de la forme amastigote de *L. infantum* après sa mise en contact avec les macrophages des chiens vaccinés, la production de NO et la présence de l'enzyme NO synthase inductible (iNOS) ont été mesurées dans le surnageant des cellules (macrophages et lymphocytes) co-cultivées.

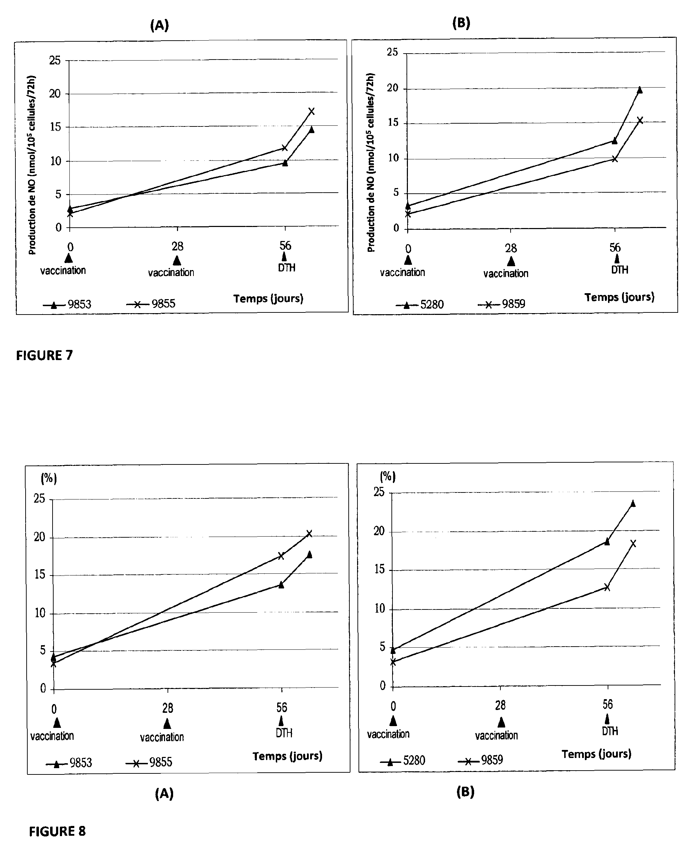

Les résultats sont représentés aux Figures 7 et 8. Seules les co-cultures des cellules des chiens vaccinés présentaient une amélioration de la production de NO. Cette augmentation est corrélée avec une augmentation de la concentration de l'enzyme NO synthase inductible (iNOS).

Comptage des cellules T CD4⁺CD5⁺ et CD8⁺CD5⁺, et des cellules B Le nombre de cellules T auxiliaires (CD4⁺CD5⁺), de cellules T cytotoxiques (CD8⁺CD5⁺) et de lymphocytes B (cellules CD21) a été évalué avant et après la vaccination par cytométrie. Le nombre de cellules sanguines, de cellules cytotoxiques et de lymphocytes B est resté stable pour tous les animaux (vaccinés et non vaccinés) au cours de l'étude. Un des deux chiens vaccinés avec la composition vaccinale LSH304 et les deux chiens vaccinés avec la composition vaccinale LSH306, ont montré une augmentation légère mais non-significative du nombre de cellules T auxiliaires, après la 2^{nd} vaccination.

### 2.2.2 Résultats durant la « phase DTH »

### Examen clinique général

Aucune réaction générale n'a été observée après l'injection de la leishmanine chez les chiens vaccinés avec la composition vaccinale LSH304 ou LSH306. Tous les animaux ont continué à prendre du poids (entre + 4% et + 7% du poids corporel) entre J56 et J63.

### Examen clinique local

Les deux chiens vaccinés avec la composition vaccinale LSH304 présentaient, au niveau du point d'injection de la leishmanine, une induration associée à une douleur et un prurit, qui ont disparu en 4 jours.

Les deux chiens vaccinés avec la composition vaccinale LSH306 présentaient, au niveau du point d'injection de la leishmanine, une induration sans douleur et un prurit, qui ont disparu en 4 jours.

En revanche, aucun des chiens vaccinés n'a montré de réaction locale au niveau du point d'injection de la solution contrôle. Par ailleurs, les 2 chiens du groupe contrôle présentaient seulement un érythème au niveau du point d'injection de la leishmanine.

Les réactions légères observées dans le groupe contrôle sont la conséquence normale d'une injection intradermique de 100 µl d'une solution d'antigènes, alors que les réactions sévères dans le groupe vacciné sont le signe d'une réponse immunitaire à médiation cellulaire spécifique d'un antigène qui est dirigée contre la leishmanine injectée.

### Analyse sérologique

Les résultats sont représentés aux Figures 4 et 5. L'injection de la leishmanine ne modifie pas le titre des anticorps IgG1 ou IgG2 anti-ESPs chez les chiens vaccinés avec la composition vaccinale LSH304. En revanche, l'injection de la leishmanine a provoqué une légère augmentation du niveau des anticorps IgG1 ou IgG2 anti-ESPs chez les chiens vaccinés avec la composition vaccinale LSH306.

### Activité leishmanicide des macrophages

La capacité des macrophages à tuer les formes amastigotes de L. *infantum* a été augmentée après injection de la leishmanine (voir Figure 6). Ce résultat contraste avec l'absence d'activité leishmanicide des macrophages du groupe contrôle tout au long de l'étude. Cette augmentation de l'activité leishmanicide des macrophages est corrélée avec une augmentation de la production de NO ainsi que la présence de NO synthase inductible (iNOS) (voir Figures 7 et 8).

### 2.2.3 Conclusion

Il ressort de cette étude, que le meilleur rapport entre l'efficacité et l'innocuité est observé avec la composition vaccinale LSH306 (contenant 60 µg de QA21). En effet, une réaction locale a été observée seulement pour un chien après la 2^{nd} vaccination. En outre, aucune hyperthermie ou dépression n'a été observée. La réponse humorale anti- ESPs était bien développée. Les macrophages des chiens vaccinés avec cette composition vaccinale étaient capables de tuer les amastigotes de *L infantum* grâce à l'activation de l'enzyme iNOS (qui produit du NO). En outre, les deux chiens vaccinés ont développé une réaction intradermique 48 heures après l'injection de la leishmanine, ce qui est le signe d'une réponse immunitaire à médiation cellulaire.

## Revendications

1. Composition vaccinale destinée à la prévention ou au traitement de la leishmaniose chez un mammifère de l'espèce *Canis lupus*, **caractérisée en ce qu'**elle est constituée :
- d'une quantité de protéines d'excrétion-sécrétion (ESPs) issues de promastigotes et/ou d'amastigotes de *Leishmania* sp., produites dans un milieu axénique et asérique, comprise entre 80 et 140 µg par dose vaccinale ;
- d'une quantité de saponine QA21 purifiée de 60 µg par dose vaccinale; et
- optionnellement, d'un excipient pharmaceutiquement acceptable.

2. Composition selon la revendication 1, **caractérisée en ce que** la saponine QA21 purifiée est le seul adjuvant saponine.

3. Composition selon l'une quelconque des revendications 1 et 2, **caractérisée en ce que** la saponine QA21 purifiée est le seul adjuvant.

4. Composition selon l'une quelconque des revendications 1 à 3, **caractérisée en ce que** la quantité d'ESPs est de 110 µg/dose vaccinale.

5. Composition selon l'une quelconque des revendications 1 à 4, **caractérisée en ce que** ledit excipient comprend ou est constitué d'un ou plusieurs des composés choisis dans le groupe constitué par le chlorure de sodium, les agents de lyophilisation, les tampons, les conservateurs et les stabilisants.

6. Composition selon la revendication 5, **caractérisée en ce que** ledit excipient comprend du Tris (trishydroxyméthylaminométhane), du mannitol et du saccharose.

7. Composition selon l'une quelconque des revendications 1 à 6, **caractérisée en ce que** lesdites protéines d'excrétion-sécrétion sont issues de promastigotes de *Leishmania* sp.

8. Procédé de fabrication d'une composition vaccinale destinée à la prévention ou au traitement de la leishmaniose chez un mammifère de l'espèce *Canis lupus,* **caractérisé en ce qu'**il comprend le mélange :
- de protéines d'excrétion-sécrétion (ESPs) issues de promastigotes et/ou d'amastigotes de *Leishmania* sp., produites dans un milieu axénique et asérique, comprises entre 80 et 140 µg par dose vaccinale et
- d'une quantité de saponine QA21 purifiée de 60 µg par dose vaccinale, et
- optionnellement, d'un excipient pharmaceutiquement acceptable.

9. Composition selon l'une quelconque des revendications 1 à 7, pour utilisation dans le traitement ou la prévention de la leishmaniose chez un mammifère de l'espèce *Canis lupus.*

## Patentansprüche

1. Impfzusammensetzung, die zur Vorbeugung oder zur Behandlung von Leishmaniose bei einem Säugetier der Spezies *Canis lupus* bestimmt ist, **dadurch gekennzeichnet, dass** sie aus Folgendem besteht:
- aus einer Menge Exkretions-Sekretionsproteine (ESP), die von Promastigoten und/oder Amastigoten von *Leishmania sp.* stammen, die in einem axenischen und serumfreien Medium hergestellt wurden, die zwischen 80 und 140 µg pro Impfdosis enthalten ist;
- aus einer Menge gereinigtes Saponin QA21 von 60 µg pro Impfdosis; und
- wahlweise aus einem pharmazeutisch annehmbaren Träger.

2. Zusammensetzung nach Anspruch 1, **dadurch gekennzeichnet, dass** das gereinigte Saponin QA21 das einzige Adjuvans auf Saponinbasis ist.

3. Zusammensetzung nach einem der Ansprüche 1 und 2, **dadurch gekennzeichnet, dass** das gereinigte Saponin QA21 das einzige Adjuvans ist.

4. Zusammensetzung nach einem der Ansprüche 1 bis 3, **dadurch gekennzeichnet, dass** die Menge an ESP 110 µg/Impfdosis beträgt.

5. Zusammensetzung nach einem der Ansprüche 1 bis 4, **dadurch gekennzeichnet, dass** der Träger eine oder mehrere der Verbindungen, die aus der Gruppe gewählt sind, die aus Natriumchlorid, Lyophilisierungsmitteln, Puffern, Konservierungsstoffen und Stabilisationen besteht, umfasst oder daraus gebildet ist.

6. Zusammensetzung nach Anspruch 5, **dadurch gekennzeichnet, dass** der Träger Tris (Trishydroxymethylaminomethan), Mannitol und Saccharose umfasst.

7. Zusammensetzung nach einem der Ansprüche 1 bis 6, **dadurch gekennzeichnet, dass** die Exkretions-Sekretionsproteine von Promastigoten von *Leishmania sp.* stammen.

8. Verfahren zur Herstellung einer Impfzusammensetzung, die zur Vorbeugung oder zur Behandlung von Leishmaniose bei einem Säugetier der Spezies *Canis lupus* bestimmt ist, **dadurch gekennzeichnet, dass** sie eine Mischung umfasst, die aus Folgendem besteht:
- Exkretions-Sekretionsproteinen (ESP), die von Promastigoten und/oder Amastigoten von *Leishmania sp.* stammen, die in einem axenischen und serumfreien Medium hergestellt wurden, die zwischen 80 und 140 µg pro Impfdosis enthalten sind und
- aus einer Menge gereinigtes Saponin QA21 von 60 µg pro Impfdosis, und
- wahlweise aus einem pharmazeutisch annehmbaren Träger.

9. Zusammensetzung nach einem der Ansprüche 1 bis 7 zur Verwendung bei der Behandlung oder der Vorbeugung von Leishmaniose bei einem Säugetier der Spezies *Canis lupus.*

## Claims

1. A vaccine composition for preventing or treating leishmaniasis in a mammal of the species *Canis lupus,* **characterized in that** it consists:
- of an amount of excretory-secretory proteins (ESPs) from promastigotes and/or amastigotes of *Leishmania* sp., produced in an axenic, serum-free medium, comprised between 80 and 140 µg per vaccine dose;
- of an amount of purified QA21 saponin of 60 µg per vaccine dose; and
- optionally, of a pharmaceutically acceptable excipient.

2. The composition as claimed in claim 1, **characterized in that** the purified QA21 saponin is the only saponin adjuvant.

3. The composition as claimed in any one of claims 1 and 2, **characterized in that** the purified QA21 saponin is the only adjuvant.

4. The composition as claimed in any one of claims 1 to 3, **characterized in that** the amount of ESPs is 110 µg/vaccine dose.

5. The composition as claimed in any one of claims 1 to 4, **characterized in that** said excipient comprises or consists of one or more of the compounds chosen from the group consisting of sodium chloride, lyophilization agents, buffers, preservatives and stabilizers.

6. The composition as claimed in claim 5, **characterized in that** said excipient comprises Tris (trishydroxymethylaminomethane), mannitol and sucrose.

7. The composition as claimed in any one of claims 1 to 6, **characterized in that** said excretory/secretory proteins are from promastigotes of *Leishmania* sp.

8. A method for manufacturing a vaccine composition for preventing or treating leishmaniasis in a mammal of the species *Canis lupus,* **characterized in that** it comprises the mixing:
- of excretory/secretory proteins (ESPs) from promastigotes and/or amastigotes of *Leishmania* sp., produced in an axenic, serum-free medium, comprised between 80 and 140 µg per vaccine dose and
- of an amount of purified QA21 saponin of 60 µg per vaccine dose, and
- optionally, of a pharmaceutically acceptable excipient.

9. The composition as claimed in any one of claims 1 to 7, for use in treating or preventing leishmaniasis in a mammal of the species *Canis lupus.*
